(19) Europäisches Patentamt
European Patent Office
Office européen des brevets

(11) Veröffentlichungsnummer: **0 245 669 B1**

(12) **EUROPÄISCHE PATENTSCHRIFT**

(45) Veröffentlichungstag der Patentschrift: **01.12.93**

(21) Anmeldenummer: **87105633.9**

(22) Anmeldetag: **15.04.87**

(51) Int. Cl.[5]: **A61K 33/30**, A61K 31/19, A61K 31/315, A61K 31/28, A61K 33/24, A61K 33/00, A61K 7/42, //(A61K33/30,33:24, 33:00,31:315,31:19,31:13, 31:155,31:195,31:185,31:66)

# (54) Pharmazeutisches Präparat zur Verhütung der Schädigung lebender Zellen durch freie Radikale, beziehungsweise zur Erhöhung der Wirksamkeit von organischen Schwefelverbindungen und Verfahren zur Erhöhung der Lebensdauer isolierter Organe.

(30) Priorität: **14.05.86 CH 1969/86**

(43) Veröffentlichungstag der Anmeldung:
**19.11.87 Patentblatt 87/47**

(45) Bekanntmachung des Hinweises auf die Patenterteilung:
**01.12.93 Patentblatt 93/48**

(84) Benannte Vertragsstaaten:
**BE CH DE FR GB IT LI LU NL SE**

(56) Entgegenhaltungen:
**EP-A- 0 073 502**
**DE-B- 1 095 463**
**US-A- 3 345 415**
**CH-A- 641 045**
**US-A- 3 335 054**
**US-A- 4 167 564**

**Burger's medicinal chemistry, 4(111) (1981), 34**

(73) Patentinhaber: **Floersheim, Georg L. Prof. Dr.**
**Lange Gasse 88**
**CH-4052 Basel(CH)**

(72) Erfinder: **Floersheim, Georg L. Prof. Dr.**
**Lange Gasse 88**
**CH-4052 Basel(CH)**

(74) Vertreter: **Blum, Rudolf Emil Ernst et al**
**c/o E. Blum & Co**
**Patentanwälte**
**Vorderberg 11**
**CH-8044 Zürich (CH)**

EP 0 245 669 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

### HINTERGRUND DER ERFINDUNG

Es ist bekannt, dass freie Radikale lebende Zellen schädigen, und beispielsweise werden die Schäden, die nach einer Bestrahlung mit ionisierenden Strahlen beobachtet werden, auf das Auftreten von freien Radikalen zurückgeführt. Es ist ferner bekannt, dass Schwefel enthaltende organische Verbindungen bei prophylaktischer Verabreichung das Auftreten derartiger Schäden verhindern können, und Schwefel enthaltende Verbindungen werden ferner auch auf anderen therapeutischen Gebieten, beispielsweise zur Behandlung von Leberschäden, herangezogen.

Die Hauptnachteile der bisher verwendeten Schwefelverbindungen ist ihre rasche Wirkungsabnahme, sodass sie beispielsweise unmittelbar vor einer zu erwartenden ionisierenden Bestrahlung verabreicht werden müssen und ferner ihre hohe Toxizität.

Des weiteren sind pharmazeutische Präparate bekannt, beispielsweise Aufbaupräparate, welche metallische Spurenelemente, einschliesslich Zinkverbindungen enthalten und welche zur Verhinderung von Mangelerscheinungen verabreicht werden (US-A-4167564).

Ziel der vorliegenden Erfindung war es, Wirkstoffe mit geringerer Toxizität als die bisher zu diesem Zweck eingesetzten Schwefelverbindungen zu entwickeln, welche zur Herstellung von pharmazeutischen Präparaten zur Verhütung der Schädigung lebender Zellen von Säugern durch freie Radikale verwendet werden können, beziehungsweise durch welche die Wirkung von pharmazeutischen Präparaten auf Basis von organischen Schwefelverbindungen synergistisch gesteigert werden kann.

Ein weiteres Ziel der vorliegenden Erfindung war es, ein Verfahren zur Erhöhung der Lebensdauer isolierter Organe zu entwickeln, die von einem Perfusionsmedium durchspült werden.

### BESCHREIBUNG DES STANDES DER TECHNIK

In der Schweizer Patentschrift Nr. 641 045 wird ein pharmazeutisches Präparat zur Heilung und Vorbeugung von Geschwulstkrankheiten beschrieben, das zwingendermassen eine anorganische Komponente und ausserdem insgesamt sechs unterschiedliche organische Komponenten enthalten muss. Die anorganische Komponente umfasst elf Elemente, nämlich Bor, Fluor, Magnesium Vanadium, Mangan, Eisen, Kobalt, Nickel, Kupfer, Zink und Molybdän in einer wasserlöslichen Form und die in der wässrigen Lösung sauer oder neutral reagieren, wobei die in dieser anorganischen Komponente enthaltenen Zinkverbindungen vorzugsweise als Sulfat oder dessen Hydrat eingesetzt werden. Die entsprechenden Präparate wurden an krebskranke Patienten verabreicht, denen durch chirurgische oder radiologische Behandlung oder die Verabreichung von Cytostatica nicht mehr geholfen werden konnte.

In den USA Patentschriften Nr. 3 345 415 und 3 335 054 werden Strahlenschutzpräparate beschrieben, welche als Wirkstoff Alkylaminoäthanthiole enthalten, in welchen die Alkylgruppe geradkettig ist und 8 - 10 Kohlenstoffatome aufweist. Schon früher sind bestimmte Aminoäthanthiole, zum Beispiel Cystein, 2-Aminoäthanthiol, 2-Mercaptoäthylguanidin und ähnliche als Strahlenschutzmittel eingesetzt worden und es wurde bereits damals angenommen, dass die Schädigungen, die durch ionisierende Strahlen hervorgerufen werden, auf die schädigende Wirkung von dabei gebildeten freien Radikalen auf den Zellmechsnismus zurückzuführen sind.

In der deutschen Auslegeschrift 1 095 463 werden. Strahlenschutzpräparate beschrieben, die Salze der Aminosäuren Cystin und Methonin mit dreiwertigen seltenen Erden, insbesondere den seltenen Erden Cer, Lanthan, Praseodym und Neodym enthalten.

Auch in der Veröffentlichung "Radiation Protection and Recovery" (Herausgeber A. Hollander, Verlag Pergamon Press, Oxford, 1960) wird im Kapitel von D.G. Doherty (Seite 45 - 86) eine prophylaktische Behandlung von Säugetieren gegen die Einwirkung von ionisierenden Strahlen durch die prophylaktische Verabreichung von entsprechenden Wirkstoffen erläutert. Bisher wurden zu diesem Zwecke hauptsächlich geschützte Thiole oder freie Thiole verwendet, wie zum Beispiel Cystein und Cysteinderivate (siehe die Veröffentlichung von H.M. Patt et al. in "Science", Band 110, Seite 213, 1949), $\beta$-Mercaptoäthylamin (siehe die Veröffentlichung von Z.M. Bacq et al. in "Science" Band 117, Seite 633, 1953), Glutathion (siehe die Veröffentlichung von W.H. Chapman et al. in "Proc. Soc. Exp. Biol. Med." Band 75, Seite 318, 1950), S,2-Aminoäthylisothioharnstoff (siehe die Veröffentlichung von D.G. Doherty et al. in "Proc. Soc. Exp. Biol. Med." Band 89, Seite 312, 1955) und eine Reihe weiterer Thiolverbindungen eingesetzt.

Bei diesen bisher zur prophylaktischen Behandlung bei einer zu erwartenden Bestrahlung mit ionisierenden Strahlen eingesetzten Wirkstoffen traten Probleme bezüglich einer adäquaten Dosierung auf, weil zur Erzielung einer entsprechenden Strahlenschutzwirkung hohe Dosierungen angewandt werden mussten,

die in der Nähe derjenigen Dosierungen lagen, wo die fraglichen Thiole bereits toxische Wirkungen zeigten.

Es sei in diesem Zusammenhang ebenfalls auf die bereits oben genannte Veröffentlichung von D.G. Doherty im Band "Radiation Protection and Recovery" hingewiesen.

Ein weiterer Nachteil der bei der Anwendung von organischen Schwefelverbindungen, beispielsweise Thiolen und Thiolderivaten, zur prophylaktischen Behandlung bei einer zu erwartenden Bestrahlung mit ionisierenden Strahlen auftritt, ist derjenige, dass diese Schwefelverbindungen ganz knapp vor der zu erwartenden Bestrahlung verabreicht werden müssen, weil sie ihre Strahlenschutzwirkung im lebenden Organismus nur während kurzer Zeiträume aufweisen. Der optimale Schutz gegen die Einwirkung von ionisierenden Strahlen kann mit den bisher zu diesem Zweck eingesetzten organischen Schwefelverbindungen im allgemeinen nur dann erzielt werden, wenn diese unmittelbar vor der zu erwartenden Bestrahlung mit ionisierenden Strahlen durch Injektion verabreicht werden. Im allgemeinen haben diese Verbindungen bereits 30 Minuten nach der Verabreichung einen Teil ihrer Wirksamkeit verloren und sie sind 120 Minuten nach der Verabreichung nicht mehr wirksam.

Es ist ferner bereits bekannt, Schwermetalle und deren Komplexe in pharmazeutischen Präparaten zu verwenden, um eine ausreichende Versorgung von tierischen Organismen mit den entsprechenden Schwermetallen zu gewährleisten. So sind beispielsweise entsprechende Präparate, die Zinksalze, wie Zinkaspartat, enthalten, im Handel erhältlich und in der USA Patentschrift Nr. 4 167 564 werden stabilisierte Komplexe aus Metall und Proteinen beschrieben, die Metall-Koordinationskomplexe der Metalle Eisen, Kupfer, Zink, Mangan, Kobalt, Chrom, Calcium, Magnesium und Vanadin mit Aminosäuren, einschliesslich Aspartinsäure und Schwefel enthaltende Aminosäuren, wie Cystin oder Cystein sind. Auch diese Metallkomplexe werden zur Erhöhung der Aufnahme der entsprechenden Mineralstoffe durch den Organismus warmblütiger Tiere eingesetzt.

In Burger's Medicinal Chemistry, 4 (III) (1981), Seite 34, wird die Rolle von Metallionen im Strahlenschutz beschrieben. Es zeigte sich dabei, dass bei der Bestrahlung von getrockneten Samen die Schädigung durch die Anwesenheit von $Cu^{2+}$ Ionen erhöht, jedoch durch die Anwesenheit von $Fe^{2+}$, $Zn^{2+}$, $Mn^{2+}$ und $Mg^{2+}$ Ionen vermindert wird (siehe rechte Spalte, vorletzter Absatz). Da jedoch im nachfolgenden Absatz darauf hingewiesen wird, dass $Cu^{2+}$ Ionen und ebenfalls $Fe^{2+}$ Ionen zu einer Erniedrigung der Radikalkonzentrationen von Trypsin und Cystein-Schwefelradikalen führen, lässt diese Veröffentlichung darauf schliessen, dass bei pflanzlichen Zellen kein Zusammenhang zwischen Senkung der Radikale und Strahlenschutzwirkung anzutreffen ist.

Es wurde jetzt überraschenderweise gefunden, dass wasserlösliche Zinkssalze oder wasserlösliche Zinkkomplexe mit einer anorganischen oder organischen Säure, insbesondere Zinksalze oder Zinkkomplexe von Aminocarbonsäuren oder Hydroxycarbonsäuren als Wirkstoff zur Herstellung von pharmazeutischen Präparaten zur Verhütung der Schädigung lebender Zellen von Säugern durch freie Radikale verwendet werden können. Die entsprechenden pharmazeutischen Präparate, die durch die erfindungsgemässe Verwendung der Zinksalze oder Zinkkomplexe herstellbar sind, weisen eine wesentlich längere Wirkungsdauer und eine bedeutend geringere Toxizität auf, als die bisher verwendeten Schwefelverbindungen, wenn sie als Wirkstoff zur Herstellung von pharmazeutischen Präparaten eingesetzt werden, die beispielsweise als prophylaktisch zu verabreichende Strahlenschutzmittel einsetzbar sind.

Des weiteren wurde überraschenderweise gefunden, dass Zinksalze und Zinkkomplexe zur Herstellung von solchen pharmazeutischen Präparaten verwendet werden können, die synergistisch mit organische Schwefelverbindungen enthaltenden pharmazeutischen Präparaten zusammenwirken und die die Wirksamkeit dieser organische Schwefelverbindungen enthaltenden Präparate deutlich erhöhen. Durch die Verwendung der Zinksalze kann beispielsweise die Wirksamkeit entsprechender Schwefel enthaltender Leberschutzpräparate oder Strahlenschutzpräparate erhöht werden.

## BESCHREIBUNG DER ERFINDUNG

Ein Gegenstand der vorliegenden Erfindung ist die Verwendung von wasserlöslichen Zinksalzen oder wasserlöslichen Zinkkomplexen mit einer anorganischen oder organischen Säure als Wirkstoff zur Herstellung eines pharmazeutischen Präparate zur Verhütung der Schädigung lebender Zellen von Säugern durch freie Radikale, wobei vorzugsweise ein Zinksalz oder ein Zinkkomplex einer Aminocarbonsäure oder einer Hydroxycarbonsäure als Wirkstoff dieses pharmazeutischen Präparates verwendet wird.

Ein weiterer Gegenstand der vorliegenden Erfindung ist die Verwendung von wasserlöslichen Zinksalzen oder wasserlöslichen Zinkkomplexen mit einer anorganischen oder organischen Säure als Wirkstoff zur Herstellung von pharmazeutischen Präparaten, welche die Wirksamkeit von pharmazeutischen Präparaten erhöhen, die organische Schwefelverbindungen enthalten, wobei eine synergistische Wirkung der genannten Zinksalze oder Zinkkomplexe mit den organischen Schwefelverbindungen auftritt.

Schliesslich betrifft die vorliegende Erfindung ein Verfahren zur Erhöhung der Lebensdauer isolierter, von einem Perfusionsmedium durchspülter Organe, wobei dieses Verfahren dadurch gekennzeichnet ist, dass man eine Schädigung der lebenden Zellen durch freie Radikale vermindert, indem man dem Perfusionsmedium ein Zinksalz oder einen Zinkkomplex zusetzt und allenfalls ausserdem eine Schwefel enthaltende organische Verbindung.

Es ist schon lange bekannt, dass bei der Einwirkung von ionisierender Strahlung, wie Röntgenstrahlen oder Gammastrahlen, und auch bei der Einwirkung von Ultraviolettstrahlung auf lebende Zellen, freie Radikale gebildet werden und dass diese ihre schädigende Wirkung auf die lebenden Zellen ausüben. Auch bei der Behandlung von Patienten mit Sauerstoff, beispielsweise einer Intensivbehandlung nach Unfällen, chirurgischen Eingriffen oder der entsprechenden Behandlung von neugeborenen Kindern mit Atmungsschwierigkeiten wurden schon lange schädliche Nebenwirkungen festgestellt, die in neuerer Zeit auf freie Radikale, wie freie Sauerstoffradikale oder Peroxidradikale zurückgeführt werden. Auch bei Gewebsalterungsvorgängen, bei Einwirkung von Toxinen oder pharmazeutischen Wirkstoffen fit toxischer Nebenwirkung wird angenommen, dass freie Radikale gebildet werden, die eine Schädigende Wirkung oder zusätzliche schädigende Wirkung ausüben. In jüngster Zeit wurden auch freie Radikale bei der neuerlichen Durchblutung von Organen nach einer vorherigen Blutleere, also Reperfusion nach einer Ischaemie festgestellt und die entsprechenden Schädigungen durch freie Radikale, können nach einer entsprechenden Durchblutungsstörung des Gehirns und der inneren Organe oder der peripheren Organe auftreten, wenn diese erneut durchblutet werden. Dies ist der Fall, wenn Blockierungen, die den Blutdurchtritt hemmen oder verhindern, mit Hilfe chemotherapeutischer Mittel oder mechanisch-chirurgischer Eingriffe beseitigt werden und dadurch eine neue Durchblutung, beispielsweise nach einem Herzinfarkt, Gehirnarterie verschluss oder Erfrierung eintritt. Die entsprechenden Schäden durch freie Radikale wurden erst in jüngster Zeit festgestellt und es sei in diesem Zusammenhang auf die Veröffentlichung von J.L. Marx mit der Titel "Oxygen Free Radicals Linked to Many Diseases" in Science, 30. Januar 1987, und die Veröffentlichung von D.M. Barnes in Science, 6. Februar 1987 hingewiesen.

Bevorzugte pharmazeutische Präparate, die durch die Verwendung der wasserlöslichen Zinksalze oder Zinkkomplexe erhältlich sind, sind daher solche an lebende Patienten zu verabreichende Präparate, welche zur Bekämpfung von durch freie Radikale ausgelöste Gewebsschäden dienen, die bei der Einwirkung einer ionisierenden Bestrahlung oder einer Ultraviolettbestrahlung, bei der neuerlichen Durchblutung von Organen nach einer vorherigen Blutleere, bei der Behandlung von Patienten mit Sauerstoff, bei Gewebsalterungsvorgängen oder bei der Einwirkung von Toxinen oder pharmazeutischen Wirkstoffen mit toxischer Nebenwirkung zu erwarten sind.

Bevorzugte derartige Präparate sind solche, die zur prophylaktischen Behandlung bei einer zu erwartenden Einwirkung von radioaktiven Stoffen verabreicht werden, beispielsweise an Personen, die nach einem Reaktorunfall Aufräumungsarbeiten leisten müssen. Ein weiteres Anwendungsgebiet derartiger Präparate ist die Verabreichung bei einer strahlentherapeutischen Behandlung von Krebserkrankungen, wobei durch das Zink oder einen zinkkomplex enthaltende Präparat das gesunde Gewebe vor der Einwirkung der ionisierenden Bestrahlung geschützt wird, nicht jedoch das durch Krebs entartete Gewebe.

Beispiele für pharmazeutische Präparate, die verabreicht werden, um durch freie Radikale ausgelöste Gewebsschäden zu verhindern, die bei der neuerlichen Durchblutung von Organen nach einer vorherigen Blutleere auftreten können, sind Präparate, die bei einer Durchblutungsstörung des Gehirnes, der inneren Organe oder der peripheren Organe vor der erneuten Durchblutung verabreicht werden. Derartige Präparate werden bei der Behandlung von Herzinfarkt oder Gehirnarterienverschluss eingesetzt, und zwar zweckmässigerweise gleichzeitig mit oder vor pharmazeutischen Präparaten, die zu einer Auslösung der die Durchblutung verhindernden oder vermindernden Koagulate dienen oder vor einer mechanisch durchgeführten Beseitigung der den Durchfluss behindernden Blockierungen.

In gleicher Weise sind die durch die erfindungsgemässe Verwendung erhältlichen Präparate bei der Behandlung von peripheren Durchblutungsstörungen einsetzbar, beispielsweise Durchblutungsstörungen der Extremitäten. Durch derartige Präparate können ferner die bei Erfrierungen auftretenden Gewebsschäden gemildert werden.

Um die Folgeschäden bei der Behandlung von Patienten mit Sauerstoff zu verhindern oder zu mildern, werden die durch die erfindungsgemässe Verwendung erhältlichen Präparate zweckmässigerweise unmittelbar vor der Behandlung mit dem Sauerstoff verabreicht. Behandlungen mit Sauerstoff sind beispielsweise bei Neugeborenen, die unter Atmungsbeschwerden leiden, bei Intensivtherapie nach Unfällen oder Operationen häufig nötig.

Auch bei der Einwirkung von ultravioletter Strahlung auf lebendes Gewebe treten Schädigungen durch freie Radikale auf und auch diese Schädigungen können mit Hilfe entsprechender Präparate gemildert werden. zweckmässigerweise werden auch hier die durch die erfindungsgemässe Verwendung erhältlichen

pharmazeutischen Präparate oral oder topisch, beispielsweise als Sonnenschutzcreme, vor einer zu erwartenden ultravioletten Bestrahlung verabreicht.

Auch solche durch freie Radikale hervorgerufene Schäden, die bei der Hautalterung und bei Hautentzündungen anzutreffen sind, können mit Hilfe der durch die erfindungsgemässe Verwendung erhältlichen pharmazeutischen Präparate wirksam verhindert oder gemildert werden, und auch in diesem Falle werden die pharmazeutischen Präparate vorzugsweise als auf die Haut auftragbare pharmazeutische Präparate oder kosmetische Präparate formuliert.

Ein weiteres Einsatzgebiet der durch die erfindungsgemässe Verwendung erhältichen pharmazeutischen Präparate ist die Behandlung von Gelenksentzündungen, insbesondere Arthritiden. Auch hier werden die entsprechenden pharmazeutischen Präparate zweckmässigerweise in Kombination mit in üblicher Weise zur Behandlung von Gelenksentzündungen eingesetzten entzündungshemmenden Mitteln verwendet, um die durch freie Radikale hervorgerufenen Schädigungen zu vermindern.

Im Zusammenhang mit Lebererkrankungen, also Hepatopatienen, wurden Schädigungen durch die Bildung von Peroxiden verschiedener Lipide beobachtet. Auch diese durch Lipidperoxidradikale hervorgerufenen Schädigungen können wirkungsvoll mit Hilfe der durch die erfindungsgemässe Verwendung erhältlichen pharmazeutischen Präparate behandelt werden.

Es ist bekannt, dass Zinkaspartat die akute Toxizität von Aethanol vermindert (siehe die Veröffentlichung von G.L. Floersheim,Agents Actions, 16, 580 [1985]) und auch die Regeneration von Leberschäden nach einer Vergiftung mit dem Knollenblätterpilz Amanita phallaoides konnte durch die Verabreichung von Zinkionen beschleunigt werden (siehe die Veröffentlichung von G.L. Floersheim et al. in Agents Actions, 14, 124 [1984]. Es zeigte sich jetzt, dass mit Hilfe der durch die erfindungsgemässe Verwendung herstellbaren pharmazeutischen Präparate ganz allgemein Schädigungen durch solche Toxine gemildert werden können, bei deren Einwirkung in den Zellen der Säuger freie Radikale gebildet werden.

Auch bei der Durchspülung von isolierten lebenden Organen mit einem Perfusionsmedium können durch freie Radikale Schädigungen der lebenden Zellen verursacht werden. Weitere durch die erfindungsgemässe Verwendung erhältliche pharmazeutische Präparate sind dementsprechend solche, die einem Perfusionsmedium zugesetzt werden, das zur Durchspülung isolierter Organe eingesetzt wird. Durch die Zugabe der Zinksalze oder Zinkkomplexe zu dem Perfusionsmedium kann die Lebensdauer der isolierten Organe deutlich erhöht werden, bzw. der Reperfusionsschaden vermindert werden.

Die Zinksalze oder Zinkkomplexe, die gemäss der erfindungsgemässen Verwendung zur Herstellung von pharmazeutischen Präparaten eingesezt werden, bzw. im erfindungsgemässen Verfahren verwendet werden, sind wasserlösliche Zinksalze mit anorganischen oder organischen Säuren oder wasserlösliche Zinkkomplexe. Speziell bevorzugt sind dabei Zinksalze oder Zinkkomplexe mit Aminosäuren, beispielsweise sauren Aminosäuren, wie Zinkaspartat oder Zinksalze mit schwefelhaltigen Aminosäuren oder Hydroxycarbonsäuren.

Es zeigte sich ferner, dass die durch die erfindungsgemässe Verwendung von Zinksalzen oder Zinkkomplexen hergestellten pharmazeutischen Präparate in der Lage sind, die Wirksamkeit von pharmazeutischen Präparaten zu steigern, welche als Wirkstoff eine Schwefel enthaltende organische Verbindung enthalten.

Eine derartige Steigerung der Wirksamkeit konnte gezeigt werden, wenn die durch die erfindungsgemässe Verwendung hergestellten pharmazeutischen Präparate vor dem die Schwefelverbindungen enthaltenden pharmazeutischen Präparat, nach dem die Schwefelverbindung enthaltenden pharmazeutischen Präparat oder gleichzeitig mit dem die Schwefelverbindung enthaltenden pharmazeutischen Präparat verabreicht wurden.

Bevorzugte, durch die erfindungsgemässe Verwendung erhaltene pharmazeutische Präparate, enthalten dementsprechend zusätzlich zu dem Zinksalz oder dem Zinkkomplex noch mindestens eine organische Schwefelverbindung oder der in den pharmazeutischen Präparaten enthaltene Zinkkomplex ist ein Komplex mit einer organischen Schwefelverbindung.

Die durch die erfindungsgemässe Verwendung erhaltenen pharmazeutischen Präparate zur Erhöhung der Wirksamkeit von organische Schwefelverbindungen enthaltenden pharmazeutischen Präparaten sind vorzugsweise ebenfalls solche Präparate, die zur Bekämpfung von Gewebsschäden dienen, die durch freie Radikale ausgelöst werden. Beispiele für derartige Präparate sind Präparate zur Bekämpfung von Schäden bei einer Einwirkung einer ionisierenden Bestrahlung, beispielsweise Präparate, die vor einer Röntgenbestrahlung zu diagnostischen Zwecken oder im Zuge einer Krebstherapie verabreicht werden, sowie Präparate zur Behandlung von Erkrankungen der Leber, zur Behandlung von Haut- und Gelenksentzündungen, zur Behandlung von altersbedingten Schäden oder Präparate, die zu den anderen bereits vorhin genannten Zwecken eingesetzt werden, einschliesslich entsprechender Präparate, die prophylaktisch verabreicht werden um die erwähnten Schäden zu vermeiden.

Die durch die erfindungsgemässe Verwendung hergestellten pharmazeutischen Präparate zur Erhöhung der Wirksamkeit von organische Schwefelverbindungen enthaltenden pharmazeutischen Präparaten enthalten als Wirkstoff wasserlösliche Zinksalze mit organischen oder anorganischen Säuren oder entsprechende Chelate der Zinkionen. Bevorzugte derartige pharmazeutische Präparate enthalten als weitere Komponente eine organische Schwefelverbindung, die mit den Zinksalzen synergistisch zusammenwirkt, wobei sich gegebenenfalls aus den genannten Zinksalzen und der Schwefelverbindung, beispielsweise einer Thiolverbindung, bei der Formulierung der Präparate bereits ein Chelatkomplex zwischen der Thiolverbindung und dem entsprechenden Zinksalz bildet.

Beispiele für Zinksalze mit anorganischen Säuren, die erfindungsgemäss als Wirkstoff bei der Herstellung der pharmazeutischen Präparate verwendet werden können, sind Chloride, Sulfate, Nitrate oder Carbonate. Beispiele für bevorzugte Zinksalze mit organischen Carbonsäuren, die als Wirkstoff zur Herstellung der pharmazeutischen Präparate verwendet werden können, sind Acetate, sowie Salze mit Aminocarbonsäuren oder Hydroxycarbonsäuren, beispielsweise Aspartate, Histidinate oder Orotate (Salze der Uracil-6-carbonsäure).

Von den Chelaten des Zinks sind diejenigen mit schwefelhaltigen organischen Verbindungen, insbesondere Thiolverbindungen, speziell bevorzugt.

Auch diejenigen pharmazeutischen Präparate, die zur Verhütung der Schädigung lebender Zellen durch freie Radikale dienen und die durch die erfindungsgemässe Verwendung der Zinksalze oder Zinkkomplexe erhalten werden, können mit Vorteil zusätzlich noch mindestens eine organische Schwefelverbindung enthalten oder der in ihnen enthaltene Zinkkomplex ist ein Komplex mit einer organischen Schwefelverbindung.

Auch bei dem Verfahren zur Erhöhung der Lebensdauer isolierter von einem Perfusionsmedium durchspülter Organe zeigte es sich, dass die Schädigung der lebenden Zellen durch freie Radikale noch wirksamer verhindert werden kann, indem man dem Perfusionsmedium sowohl ein Zinksalz oder einen Zinkkomplex als auch zusätzlich eine Schwefel enthaltende organische Verbindung zusetzt.

Organische Schwefelverbindungen, die bei diesem Verfahren zugesetzt werden können, beziehungsweise organische Schwefelverbindungen, die in den erwähnten pharmazeutischen Präparaten enthalten sein können, sind vorzugsweise solche Schwefelverbindungen, die bereits bisher als Wirkstoff in pharmazeutischen Präparaten eingesetzt wurden.

Beispiele für organische Schwefelverbindungen, die bisher zur prophylaktischen Behandlung bei einer zu erwartenden Bestrahlung mit ionisierenden Strahlen eingesetzt wurden, sind solche mit freier SH-Gruppe, beispielsweise Cystein, L-Glutathion, Cysteamin, Penicillamin oder Aminoalkylisothioharnstoff der Formel

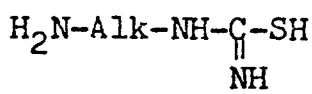

oder es sind Verbindungen mit einer geschützten SH-Gruppe, die im lebenden Organismus in eine freie SH-Gruppe verwandelt wird. Als Beispiele für bevorzugte bisher in Strahlenschutzpräparaten eingesetzten Verbindungen mit geschützer SH-Gruppe seien genannt:

das S-(2-Aminoäthyl)-isothiouroniumbromidhydrobromid der Formel

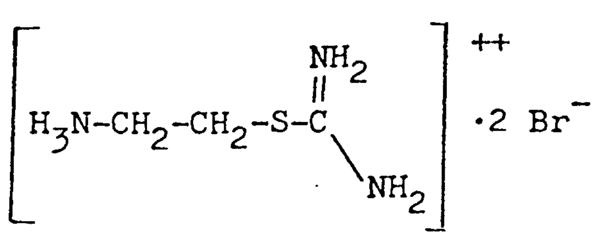

sowie Verbindungen, in denen die SH-Gruppe durch Veresterung mit Phosphorsäure oder Schwefelsäure geschützt ist, bzw.

Verbindungen, in denen die SH-Gruppe in Form einer mindestens zwei Schwefelatome umfassenden Schwefel-Schwefelbrücke geschützt ist. Als Beispiele für bisher in Strahlenschutzpräparaten eingesetzte

Verbindungen mit geschützter Gruppe seien die Verbindungen der folgenden Formeln genannt:

$HO_2\,S\text{-}(CH_2)_4\text{-}S\text{-}S\text{-}S\text{-}(CH_2)_4\text{-}SO_2H$

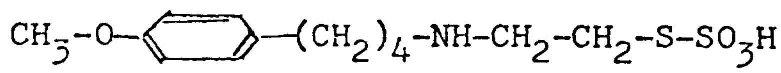

$CH_3\text{-}NH\text{-}(CH_2)_2\text{-}NH\text{-}CH_2\text{-}CH_2\text{-}CH_2\text{-}S\text{-}PO_3H$

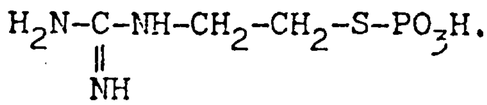

Wenn die wasserlöslichen Zinksalze oder Zinkkomplexe, erfindungsgemäss zur Herstellung von pharmazeutischen Präparaten verwendet werden, die zusätzlich bereits eine organische Schwefelverbindung enthalten, dann ist diese vorzugsweise eine solche, die bisher schon zu den angegebenen Zwecken eingesetzt wurde. Dementsprechend enthalten entsprechende Strahlenschutzpräparate als zusätzliche Komponente vorzugsweise eine der oben genannten Schwefelverbindungen, die bisher bereits in Strahlenschutzpräparaten eingesetzt wurden.

Es wurde bisher angenommen, dass pharmazeutische Präparate zur Bekämpfung von Gewebsschäden, die durch freie Radikale ausgelöst werden und die als Wirkstoff eine organische Verbindung mit freier SH-Gruppe oder geschützter SH-Gruppe enthalten, die gebildeten freien Radikale abfangen, und zwar insbesondere Sauerstoffradikale, Hydroxyradikale und Peroxyradikale der Formel $HO_2^{\cdot}$ . Es sei in diesem Zusammenhang auf die bereits weiter oben genannte Veröffentlichung von D.G. Doherty hingewiesen. Bisher wurde also angenommen, dass beispielsweise bei einer Einwirkung einer ionisierenden Strahlung die entsprechenden organische Schwefelverbindungen enthaltenden Präparate eine Schädigung durch die ionisierende Strahlung dadurch verhindern können, indem sie entweder die Anzahl an freien Radikalen vermindern, die während der Bestrahlung in den Zellen gebildet werden, oder indem sie die bei der Bestrahlung gebildeten freien Radikale abfangen und dadurch verhindern, dass sie mit solchen Molekülen oder Zellbestandteilen reagieren, die für die biologischen Reaktionen in der Zelle lebenswichtig sind, wie zum Beispiel Nukleinsäure, Proteinen und Lipiden der Zellmembran.

Es wird angenommen, dass die Zinkionen die Bildung der freien Radikale verhindern oder bereits gebildete freie Radikale abfangen und dadurch die schädigende Wirkung der freien Radikale auf die lebenden Säugerzellen beseitigen. Gegebenenfalls können dabei auch organische Schwefelverbindungen, die im lebenden Organismus vorliegen, durch Komplexbildung mit den angeführten Zinkionen in ihrer Wirkungsweise als Radikalfänger gesteigert werden.

Der Synergismus zwischen den Präparaten, die die genannten Zinksalze oder Zinkkomplexe enthalten, und den organische Schwefelverbindungen enthaltenden pharmazeutischen Präparaten, kann möglicherweise auch dadurch erklärt werden, dass die Zinkionen mit den Schwefelverbindungen, beispielsweise Thiolgruppen derselben, reagieren und so eine Stabilisierung der Schwefelverbindung erreicht wird. Dadurch können die entsprechenden Schwefelverbindungen ihre schützende Wirkung bei allen Gewebsschäden ausüben, die durch freie Radikale ausgelöst werden und möglicherweise wirken auch die in den durch die erfindungsgemässe Verwendung hergestellten synergistischen Präparate enthaltenden Zinksalze und Zinkkomplexverbindungen selbst als Radikalfänger.

Besonders ausgedehnte Untersuchungen wurden im vorliegenden Fall bezüglich synergistisch wirkenden pharmazeutischen Präpate gemacht, die Zinkionen enthalten, und zwar auf dem Gebiete des Strahlenschutzes und auch bezüglich Strahlenschutzpräparaten zur prophylaktischen Behandlung, zu deren Herstellung als Wirkstoff Zinksalze verwendet wurden. Dabei wurden auch Vergleichsversuche mit bisher bekannten Strahlenschutzpräparaten, die organische Schwefelverbindungen enthalten, durchgeführt. Es zeigte sich dabei, dass die Strahlenschutzwirkung von organische Schwefelverbindungen enthaltenden Präparaten durch die vorherige, gleichzeitige oder nachfolgende Verabreichung von Zinksalzen oder Zinkkomplexen deutlich erhöht werden kann.

Ein besonderer Vorteil der Zinksalze und Zinkkomplexe besteht darin, dass sie vom Menschen gut vertragen werden und dass die Strahlenschutzwirkung bereits bei einer tierexperimentellen Verabreichung von nur 10 bis 40 mg pro kg Körpergewicht auftritt, während die Thiolverbindungen in einer etwa achtfach so hohen Dosierung, nämlich in einer Menge von etwa 150 bis 2000 mg pro kg Körpergewicht verabreicht

werden müssen, um eine entsprechende strahlenschützende Wirkung zu erzielen. Bezüglich der guten Verträglichkeit von Zinkverbindungen beim Menschen sei auf die Veröffentlichung von P. Tschumi und G.L. Floersheim (in "Schweiz. med. Wschr.", Band 111, Seite 1573, 1981) hingewiesen.

Ein Mass für die strahlenschützende Wirkung von Strahlenschutzpräparaten ist der Dosisminderungsfaktor, der in der englischsprachigen Literatur als "dose-reduction-factor" bezeichnet wird. Es handelt sich dabei um den Quotienten der Strahlungsdosis, der ähnliche Wirkungen hervorruft, und zwar unter Verabreichung eines Strahlenschutzpräparates in der fraglichen Dosierung und ohne Verabreichung eines Strahlenschutzpräparates in der fraglichen Dosierung.

In den nachfolgenden Beispielen wurde die synergistische Wirkung der durch die erfindungsgemässe Verwendung hergestellten pharmazeutischen Präparate mit organische Schwefelverbindungen enthaltenden Präparaten anhand des Strahlenschutzes getestet und es wurde auch die Strahlenschutzwirkung der Zinkionen enthaltenden Präparate mit bisher bekannten Strahlenschutzpräparaten an Mäusen getestet. Die Versuche wurden mit weiblichen C3H Mäusen (GL. Bomholtgard, Ry, Dänemark) durchgeführt, die 20 bis 22 g wogen. Die Mäuse wurden unter Bedingungen gehalten, wo sie so viel Standard-Nagetierfutter (Nafag Pellts) fressen und so viel Leitungswasser trinken können, wie sie wollten. Die Bestrahlung erfolgte mit Gammastrahlen aus einer Kobalt-60 Quelle. Es wurden unterschiedliche Bestrahlungen vorgenommen, und zwar mit 9 Gy, 10 Gy, 11 Gy, 12 Gy, 13 Gy und 14 Gy.

10 Minuten vor der Bestrahlung, 30 Minuten vor der Bestrahlung oder 120 Minuten vor der Bestrahlung wurde ein durch die erfindungsgemässe Verwendung hergestelltes synergistisch wirkendes pharmazeutisches Präparat, bzw. ein entsprechendes, Zinkionen enthaltendes Strahlenschutzpräparat oder ein aus dem Stand der Technik bekanntes Strahlenschutzpräparat zu Vergleichszwecken durch intraperitoneale Injektion verabreicht.

Bei der Gruppe der Mäuse zu Vergleichszwecken, die ohne Verabreichung eines Strahlenschutzpräparates bestrahlt wurden, erfolgte 10 Minuten vor der Bestrahlung die Verabreichung von destilliertem Wasser. Bei diesen unbehandelten Mäusen überlebten nur 50% der Tiere eine Bestrahlung von 8 Gy, d.h. der $LD_{50}$-Wert betrug 8 Gy.

Bei der Gruppe an Mäusen, die ohne Verabreichung eines Strahlenschutzpräparates der Bestrahlung unterworfen wurden, war die letale Dosis bereits bei 9 Gy erreicht (98% der Tiere starben), während bei Bestrahlungsn mit 10 Gy, bzw. 11 Gy, keines der getesteten Tiere mehr überlebt.

Die durchgeführten Tests zeigten ferner, dass Zinksalze mit organischen Säuren eine bessere Strahlenschutzwirkung aufweisen als Zinkchlorid, bzw. Zinksulfat. Besonders vorteilhaft erwies sich dabei Zinkaspartat.

Zu Vergleichszwecken wurde ferner Kupferaspartat in einer Dosierung von 10 mg/kg Körpergewicht bis 30 mg/kg Körpergewicht und auch Natriumaspartat in einer Dosierung von 20 mg/kg Körpergewicht bis 350 mg/kg Körpergewicht getestet, wobei diese zinkfreien Salze 10 Minuten vor einer Bestrahlung mit 9 Gy injiziert wurden. Es zeigte sich dabei, dass diese zinkfreien Salze wirkungslos waren, d.h. bei einer Bestrahlung mit 9 Gy war bei den Tieren bereits die letale Dosis erreicht.

## Beispiel 1

### Strahlenschutzwirkung von Zinkaspartat

Das Zinkaspartat, nämlich Zink-bis-(DL-hydrogenaspartat) wurde in einer Dosis von 30 mg/kg Körpergewicht 30 Minuten vor der Bestrahlung und 120 Minuten vor der Bestrahlung verabreicht.

Bei einer Bestrahlung mit 11 Gy überlebten 60 % der Tiere, wenn die Verabreichung 30 Minuten vor der Bestrahlung erfolgte und 53 % der Tiere, wenn sie 120 Minuten vor der Bestrahlung erfolgte.

Bei einer Bestrahlung von 12 Gy überlebten 57 % der Tiere, wenn die Verabreichung 30 Minuten vor der Bestrahlung erfolgt war und 35 % der Tiere, wenn die Verabreichung 120 Minuten vor der Bestrahlung erfolgt war. Selbst bei einer Bestrahlung von 13 Gy überlebten 17 % der Tiere, wenn die Verabreichung 30 Minuten vor der Bestrahlung durchgeführt wurde und 14 % der Tiere, wenn die Versbreichung 120 Minuten vor der Bestrahlung durchgeführt worden war.

## Beispiel 2

### Strahlenschutzwirkung von Zinksulfat

In der gleichen Weise wie im Beispiel 1 wurde das Zinksalz in destilliertem Wasser aufgelöst und durch Injektion von 0,1. ml/10 g Körpergewicht an Mäuse intraperitoneal verabreicht. Das Zinksulfat $ZnSO_4.7\ H_2O$

wurde in einer Dosis von 22,5 mg/kg Körpergewicht, bzw. 30 mg/kg Körpergewicht verabreicht. Es zeigte sich, dass bei einer Verebreichung 10 Minuten vor der Bestrahlung in beiden Fällen 33 % der Mäuse eine Bestrahlung von 10 Gy überlebten.

Beispiel 3

Testung von Zinkchlorid

Das Zinkchlorid $ZnCl_2$ wurde, gelöst in destilliertem Wasser, wieder in einer Menge von 0,1 ml/10 g Körpergewicht intraperitoneal injiziert. Die Verabreichung erfolgte 30 Minuten vor der Bestrahlung.

Bei einer Dosierung von 10 mg/kg Körpergewicht überlebten 15 % der Mäuse eine Dosis von 10 Gy und bei einer Dosierung von 15 mg/kg Körpergewicht überlebten 20 % der Mäuse eine Dosis von 10 Gy.

Beispiel 4

In diesem Beispiel wurde die Toxizität der als Strahlenschutzmittel eingesetzten Zinksalze getestet.

Der $LD_{50}$-Wert betrug für das Zinkaspartat 55 mg/kg Körpergewicht, für das Zinkchlorid 24 mg/kg Körpergewicht und für das Zinksulfat 45 mg/kg Körpergewicht.

Beispiel 5

In diesem Beispiel zu Vergleichszwecken wurde die Strahlenschutzwirkung von Thiolgruppen haltigen bisher bekannten Strahlenschutzmitteln geprüft. Es zeigte sich dabei, dass Cystein und L-Glutathion deutlich weniger wirksam waren als die erfindungsgemäss zu verwendenden Zinkverbindungen. Bei einer Verabreichung von 600 mg/kg, 900 mg/kg und 1200 mg/kg Cystein, überlebten jeweils 3, bzw. 4, bzw. 9 von 10 Mäusen eine Bestrahlung mit 10 Gy, wenn die Verabreichung 10 Minuten vor der Bestrahlung erfolgte. Bei diesen Dosierungen wurde eine Bestrahlung mit 11 Gy von praktisch keinem der Tiere überlebt. Nur zwei von 10 Mäusen überlebten nach 1200 mg/kg, verabfolgt 10 Minuten vor der Bestrahlung.

L-Glutathion wurde in 100 mg/kg Körpergewicht, bzw. 2000 mg/kg Körpergewicht, 10 Minuten vor der Bestrahlung verabreicht. Dabei überlebten eine Bestrahlung mit 9 Gy nur 3 von 10 Tieren bei der Niedrigeren Dosierung und nur 2 von 10 Tieren bei der höheren Dosierung.

Das in der Literatur beschriebene schwefelhaltige Strahlenschutzpräparat S-(2-Aminoäthyl)-isothiouroniunibromid-hydrobromid der Formel

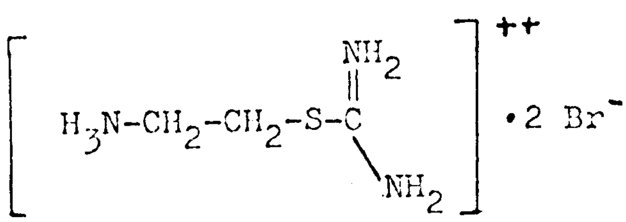

wird in der Folge mit AET abgekürzt. Es zeigte bei einer Verabreichung 10 Minuten vor der Bestrahlung dann keine Strahlenschutzwirksamkeit, wenn eine Menge von 125 mg/kg Körpergewicht gegeben wurde. In diesem Falle überlebte eine Bestrahlung mit 11 Gy, bzw. 13 Gy, kein Tier von 10 getesteten Tieren. Wurde jedoch unter sonst gleichen Bedingungen die Dosierung des AET auf 250 mg/kg Körpergewicht erhöht und erfolgte die Verabreichung wieder 10 Minuten vor der Bestrahlung, dann überlebten sämtliche von 10 getesteten Tieren eine Bestrahlung von 11 Gy, 9 von 10 getesteten Tieren eine Bestrahlung von 12 Gy und 2 von 10 getesteten Tieren eine Bestrahlung von 13 Gy. Eine Bestrahlung von 14 Gy wurde jedoch von keinem der 10 getesteten Tiere überlebt.

Cysteamin, nämlich $\beta$-Merkaptoäthylamin, zeigte keine Strahlenschutzwirkung bei einer Verabreichung 10 Minuten vor der Bestrahlung in einer Dosierung von 150 mg/kg Körpergewicht, wenn mit 11 Gy bestrahlt wurde. Hingegen ist durch eine Erhöhung der Dosierung auf 225 mg/kg Körpergewicht, wiederum 10 Minuten vor der Bestrahlung, eine recht gute Strahlenschutzwirksamkeit festgestellt worden, nämlich von den jeweils 10 getesteten Tieren überlebten 8 eine Bestrahlung mit 11 Gy 6 eine Bestrahlung mit 12 Gy, 1 eine Bestrahlung mit 13 Gy, doch keines eine Bestrahlung mit 14 Gy. Wurde jedoch die fragliche Verbindung 30 Minuten vor der Bestrahlung verabreicht, dann überlebten nurmehr 6 Tiere eine Bestrahlung mit 11 Gy und 1 Tier eine Bestrahlung mit 12 Gy.

Diese Ergebnisse zeigen sehr deutlich, dass bereits 30 Minuten nach der Verabreichung eines Strahlenschutzpräparates auf Thiolbasis die strahlenschützende Wirkung deutlich nachgelassen hat.

Nach den Arbeitsweisen der vorangegangenen Beispiele 1 bis 4, wurden weitere Tests mit den genannten weiblichen Mäusen durchgeführt, und zwar unter Verwendung der angeführten Zinksalze. Die bei diesen Tests erzielten Ergebnisse sind in der nachfolgenden Tabelle I zusammengestellt. Für jede der angewandten Bestrahlungsstärken ist in der Rubrik "Anzahl" diejenige Anzahl der Mäuse angeführt, welche die Bestrahlung überlebten, geteilt durch die Gesamtzahl der Mäuse, welche bestrahlt wurden. Ein entsprechender Wert von 1/10 bedeutet also, dass von 10 bestrahlten Mäusen eine überlebt hatte. In der Spalte mit der Bezeichnung "Prozent", ist die Prozent-Ueberlebensrate angeführt. Bei einer überlebenden Maus pro 10 getesteten Mäusen beträgt diese Ueberlebensrate 10 %.

## Tabelle I

**Ueberleben von Mäusen bei einer Bestrahlung mit 9 - 13 Gy nach einer Vorbehandlung mit Zink-aspartat**

| | Dosis mg/kg | Verabreichungszeit in Minuten vor Bestrahlung | Ueberlebensrate nach Bestrahlung mit 9 Gy Anzahl | 9 Gy % | 10 Gy Anzahl | 10 Gy % | 11 Gy Anzahl | 11 Gy % | 12 Gy Anzahl | 12 Gy % | 13 Gy Anzahl | 13 Gy % |
|---|---|---|---|---|---|---|---|---|---|---|---|---|
| Vergleich | 0 | | 1/59 | 2 | 0/73 | 0 | 0/60 | 0 | | | | |
| | 10 | -10 | 1/10 | 10 | | | | | | | | |
| | 20 | -10 | 26/39 | 67 | 13/45 | 29 | 0/20 | 0 | | | | |
| | 20 | -30 | 6/10 | 60 | 1/10 | 0 | | | | | | |
| | 20 | -120 | 5/10 | 50 | 0/10 | 0 | | | | | | |
| | 30 | -10 | 43/49 | 88 | 43/58 | 74+ | 3/30 | 10 | 1/30 | 3 | 0/20 | 0 |
| | 30 | -30 | 8/10 | 80 | 21/30 | 70 | 24/40 | 60+ | 17/30 | 57+ | 5/30 | 17 |
| | 30 | -120 | 9/10 | 90+ | 8/20 | 40 | 16/30 | 53 | 7/20 | 35 | 4/29 | 14 |
| | 40 | -10 | 17/29 | 59 | 16/29 | 55 | 1/40 | 3 | 5/30 | 17 | 1/19 | 5 |
| | 40 | -30 | 9/20 | 45 | 7/10 | 70 | 5/10 | 50 | 3/10 | 30 | 7/29 | 24+ |
| | 40 | -120 | 5/10 | 50 | 5/10 | 50 | 1/10 | 10 | 0/10 | 0 | 1/19 | 5 |

Tabelle II

Ueberlebensrate von Mäusen nach einer Vorbehandlung mit Zinkchlorid, bzw. Zinksulfat und einer Bestrahlung mit 9 - 11 Gy

| Zinksalz | Dosis mg/kg | Verabreichungszeit in Minuten vor Bestrahlung | Ueberlebensrate nach Bestrahlung mit 9 Gy Anzahl | 9 Gy % | 10 Gy Anzahl | 10 Gy % | 11 Gy Anzahl | 11 Gy % |
|---|---|---|---|---|---|---|---|---|
| Zinkchlorid | 10 | -10 | 2/10 | 20 | | | | |
| | 10 | -30 | | | 3/20 | 15 | | |
| | 15 | -10 | 3/30 | 30 | | | | |
| | 15 | -30 | | | 4/20 | 20 | | |
| | 20 | -10 | 0/10 | 10 | | | | |
| Zinksulfat | 15 | -10 | 1/10 | 0 | 2/9 | 22 | | |
| | 15 | -30 | | | | | 0/20 | 0 |
| | 22,5 | -10 | 8/10 | 80 | 3/9 | 33 | | |
| | 22,5 | -30 | | | | | 0/20 | 0 |
| | 30 | -10 | 0/10 | | 3/9 | 33 | | |
| | 30 | -30 | | | | | 1/20 | 5 |

Zu Vergleichszwecken sind in der nachfolgenden Tabelle III die Ueberlebensraten von Mäusen zusammengestellt, die nach dem in Beispiel 5 beschriebenen Verfahren einer Vorbehandlung mit einem bekannten schwefelhaltigen Strahlenschutzmittel unterworfen worden waren. Orientierende Vorversuche hatten gezeigt, dass diese Strahlenschutzmittel bereits ihre Wirkung verloren hatten, wenn sie zwei Stunden vor der Einwirkung der Bestrahlung verabreicht worden waren. Die Bestrahlung wurde, wie aus der Tabelle ersichtlich ist, zwei Minuten nach der Verabreichung, in den meisten Fällen 10 Minuten nach der Verabreichung, aber auch 30 Minuten, bzw. 120 Minuten nach der Verabreichung durchgeführt. Die Werte deuten darauf hin, dass 30 Minuten nach der Verabreichung die Schutzwirkung schon wieder deutlich

nachgelassen hat. Für jede Bestrahlungsstärke ist wiederum die Anzahl der Tiere angeführt, die nach 30 Tagen überlebt haben, geteilt durch die Gesamtzahl der Tiere, die behandelt und bestrahlt worden waren.

Tabelle III

Ueberlebensrate von Mäusen nach einer Vorbehandlung mit Thiolen und Bestrahlung

| Schwefel-verb. | Dosis mg/kg | Anwendung in Minuten vor Bestrahlung | Ueberlebensrate nach Bestrahlung mit 9 Gy | 10 Gy | 11 Gy | 12 Gy | 13 Gy | 14 Gy |
|---|---|---|---|---|---|---|---|---|
| Vergleich | 0 | | 0/10 | 0/10 | 0/10 | 0/10 | 0/10 | |
| Cysteamin | 75 | -10 | | 0/10 | | | | |
| | 150 | -10 | 10/10 | 9/10 | 0/10 | | | |
| | 225 | -2 | | | | | | 0/10 |
| | 225 | -10 | | 10/10+ | 8/10+ | 6/10+ | 1/10+ | 0/10 |
| | 225 | -30 | | | 6/10 | 1/10 | | |
| | 225 | -120 | | | 0/10 | | | |
| Cystein | 300 | -10 | 0/10 | | | | | |
| | 600 | -10 | 7/10 | 3/10 | | | | |
| | 900 | -10 | 10/10+ | 4/10 | 0/10 | | | |
| | 1200 | -10 | | 9/10+ | 2/10+ | 1/10+ | | |
| | 1200 | -30 | | | 0/10 | | | |

Tabelle III (Fortsetzung)

Ueberlebensrate von Mäusen nach einer Vorbehandlung mit Thiolen und Bestrahlung

| Schwefel-verb. | Dosis mg/kg | Anwendung in Minuten vor Bestrahlung | Ueberlebensrate nach Bestrahlung mit 9 Gy | 10 Gy | 11 Gy | 12 Gy | 13 Gy | 14 Gy |
|---|---|---|---|---|---|---|---|---|
| L-Glutathion | 1000 | -10 | 3/10 | | | | | |
| | 2000 | -10 | 2/10 | | | | | |
| AET | 30 | -10 | 1/10 | | | | | |
| | 125 | -10 | | | 0/10 | | 0/10 | |
| | 250 | -10 | | | 10/10+ | 9/10+ | 2/10+ | 0/10 |
| | 250 | -2 | | | | | 0/10 | |
| | 250 | -30 | | | 10/10 | 2/10 | | |
| | 250 | -120 | | | 0/10 | | | |

Beispiel 6

Anhand dieses Beispiels soll die synergistische Wirkung von Zinkionen und Schwefelverbindungen bezüglich eines Strahlenschutzes erläutert werden. Es wurde wieder in der gleichen Weise gearbeitet, wie

in den vorangegangenen Beispielen 1 bis 3, bzw. 5, und es wurde auch die gleiche Art an weiblichen Mäusen getestet. Die Verabreichung der Zinkionen enthaltenden Lösung und der Lösung der Schwefelverbindung erfolgte entweder nacheinander oder gleichzeitig durch intraperitoneale Injektion.

Die getesteten Schwefelverbindungen waren diejenigen, die bei alleiniger Anwendung zu den besten Ergebnissen bezüglich des Strahlenschutzes führten, nämlich Cysteamin und S-(2-Aminoäthyl)-isothiouroniumbromidhydrobromid, abgekürzt als AET.

Wie man aus den vorangegangenen Tabellen I und III sieht, überlebten bei der alleinigen Verabreichung von Schwefelverbindungen keine Mäuse eine Bestrahlungsdosis von 14 Gy und weitere Versuche, die mit Zinkaspartat alleine durchgeführt wurden, zeigten auch, dass bei einer alleinigen Verabreichung von Zinkaspartat in einer Dosierung von 20 mg/kg Körpergewicht und 30 mg/kg Körpergewicht, die Mäuse eine Bestrahlung von 14 Gy nicht überlebten.

Im Gegensatz dazu konnte bei der sequentiell kombinierten Verabreichung von 20 mg Zinkaspartat pro kg Körpergewicht und 125 mg AET pro kg Körpergewicht eine Ueberlebensrate von 90 % oder über 90 % festgestellt werden. Vergleichbare Ergebnisse wurden erzielt, wenn nicht getrennte Lösungen von Zinkaspartat und AET, sondern eine Lösung verabreicht wurde, die sowohl Zinkaspartat als auch AET in solchen Mengen enthielt, dass sie zur Verabreichung von 20 mg Zinkaspartat pro kg Körpergewicht und 125 mg AET pro kg Körpergewicht geeignet war.

Aufgrund der guten Ergebnisse bei einer Bestrahlung mit 14 Gy wurde die Bestrahlungsdosis unter den gleichen Bedingungen noch weiter gesteigert. Dabei zeigte es sich, dass überraschenderweise nach der Verabreichung von Zinkaspartat und AET in den angegebenen Mengen die Mäuse bei einer Bestrahlung mit 15 Gy und bei einer Bestrahlung mit 16 Gy immer noch eine Ueberlebensrate von 90 % zeigten. Sogar bei einer Bestrahlung mit 17 Gy betrug in diesem Falle die Ueberlebensrate noch 60 % und bei einer Bestrahlung mit 18 Gy betrug die Ueberlebensrate immer noch 30 %.

Erst eine Bestrahlung mit 19 Gy war nach dieser Vorbehandlung bei allen Mäusen letal.

Bei der Bestrahlungsdosis von 14 Gy wurde ferner überprüft, ob höhere Dosierungen an Zinkespartat plus AET zu besseren Ergebnissen führen könnten. Dabei zeigte es sich überraschendenweise, dass eine Verabreichung von 30 mg Zinkaspartat pro kg Körpergewicht + 250 AET pro kg Körpergewicht zu schlechterem Strahlenschutz führte als die Verabreichnung von 20 mg Zinkaspartat pro kg Körpergewicht plus 125 mg AET pro kg Körpergewicht.

In allen Fällen wurde die Ueberlebensrate 30 Tage nach der Einwirkung der Bestrahlung bestimmt.

In der Zeichnung ist auf der Ordinate die Ueberlebensdauer in % 30 Tage nach der Bestrahlung aufgetragen. Auf der Abszisse ist die Bestrahlungsdosis in Gy angeführt. Ein Gy entspricht 100 r.

Ferner sind die getesteten Werte für die Ueberlebensrate bei der jeweiligen Bestrahlung in den Kurven angegeben.

Die jenige Kurve, in welcher die Messpunkte mit + angeführt sind, veranschaulicht die Vergleichsversuche, d.h. in diesem Fall wurde ohne Vorbehandlung bestraht. Dabei war die Ueberlebensrate bei einer Strahlendosis von nur 7 Gy 90 %, bei 8 Gy 53 % und bei 9 Gy nurmehr 2 %. Die besten Ergebnisse, die bei der alleinigen Verabreichung von Zirkaspartat erzielt wurden, sind in der nachfolgenden Tabelle zusammengestellt.

| Bestrahlungsdosis in Gy | Ueberlebensrate in % |
|---|---|
| 8 | 100 |
| 9 | 90 |
| 10 | 74 |
| 11 | 60 |
| 12 | 57 |
| 13 | 24 |
| 14 | 0 |

Es handelt sich dabei in Fig. 1 um diejenige Kurve, deren Messpunkte mit nicht ausgefüllten Dreiecken veranschaulicht sind.

In der nachfolgenden Tabelle sind diejenigen Werte zusammengestellt, die bei der alleinigen Verabreichung von AET in optimaler Dosierung erzielt wurden.

| Bestrahlungsdosis in Gy | Ueberlebensrate in % |
|---|---|
| 11 | 100 |
| 12 | 90 |
| 13 | 20 |
| 14 | 0 |

Diese Ergebnisse veranschaulicht in der Fig. 1 diejenige Kurve, deren Messpunkte durch unausgefüllte Kreise veranschaulicht sind.

Diejenigen Werte, die bei der gleichzeitigen Verabreichung von Zinkaspartat und AET in optimaler Dosierung erzielt wurden, sind in der nachfolgenden Tabelle zusammengestellt, wobei wieder die Ueberlebensrate nach 30 Tagen bestimmt wurde.

| Bestrahlungsdosis in Gy | Ueberlebensrate in % |
|---|---|
| 13 | 100 |
| 14 | 95 |
| 15 | 90 |
| 16 | 90 |
| 17 | 60 |
| 18 | 30 |
| 19 | 0 |

Diese Ergebnisse sind in Figur 1 durch die Kurve veranschaulicht, deren Messpunkte durch ausgefüllto Kreise angezeigt sind.

In der nachfolgenden Tabelle IV sind weitere Ergebnisse zusammengestellt, die bei einer Bestrahlung der Mäuse mit 13 Gy bis 18 Gy nach einer Vorbehandlung mit Zinkaspartat plus AET erzielt wurden.

Tabelle IV

Ueberleben von Mäusen bei einer Bestrahlung mit 13 bis 18 Gy nach einer Vorbehandlung mit Zink-aspartat + AET

| Zink-aspartat | | AET | | Ueberlebensrate nach Bestrahlung mit | | | | | |
|---|---|---|---|---|---|---|---|---|---|
| Dosis mg/kg | Verabreichungszeit in Minuten vor Bestrahlung | Dosis mg/kg | Verabreichungszeit in Minuten vor Bestrahlung | 13 Gy | 14 Gy | 15 Gy | 16 Gy | 17 Gy | 18 Gy |
| 10 | -30 | 62,5 | -10 | | 0/5 | | | | |
| 10 | -30 | 125 | -10 | | 8/15 | 1/10 | 0/10 | | |
| 10 | -30 | 250 | -10 | | 10/15 | 7/10 | 1/10 | | |
| 20 | -30 | 62,5 | -10 | | 5/15 | 4/10 | 0/10 | | |
| 20 | -30 | 125 | -10 | 10/10+ | 19/20+ | 0/10 | 1/10 | 0/10 | |
| 20 | -30 | 250 | -10 | 7/10 | 15/20 | 8/10 | 5/10 | 2/10 | 0/10 |
| 30 | -30 | 62,5 | -10 | | 14/15 | 9/10+ | 3/10 | | |
| 30 | -30 | 125 | -10 | 8/10 | 15/20 | 7/10 | 8/10 | 6/10+ | 3/10+ |
| 30 | -30 | 250 | -10 | 0/10 | 2/5 | | | | |
| 30 | -120 | 250 | -10 | 5/8 | 7/15 | 5/10 | 9/10+ | 1/10 | 3/10+ |
| 30 | -10 | 250 | -10 | 5/9 | 2/5 | | | | |
| 20 | -10 | 125 | -10 | | 2/10 | | | | |
| 20 | -30 | 125 | -30 | | 0/5 | | | | |

Wenn man 225 mg/kg Körpergewicht an Cysteamin alleine 10 Minuten vor der Bestrahlung injiziert, dann tritt bei einer Bestrahlung mit 13 Gy eine Ueberlebensrate von 10 % auf. Auch hier kann die Ueberlebensrate durch die vorherige Verabreichung von Zinkaspartat gesteigert werden. Erfolgt nämlich eine Verabreichung von 30 mg Zinkaspartat pro kg Körpergewicht 30 Minuten vor der Bestrahlung und anschliessend eine Verabreichung von 225 mg/kg Körpergewicht an Cysteamin, 10 Minuten vor der Bestrahlung, dann wurde bei einer Bestrahlung mit 13 Gy eine Ueberlebensrate von 67 % festgestellt.

Beispiel 7

Es wurde in der gleichen Weise gearbeitet wie in Beispiel 6, jedoch wurde jetzt der Synergismus zwischen den Schwefelverbindungen der Formel

$HO_2S\text{-}(CH_2)_4\text{-}S\text{-}S\text{-}S\text{-}(CH_2)_4\text{-}SO_2H$

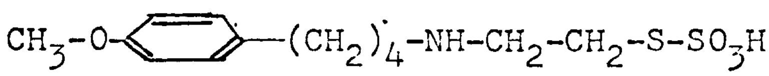

$CH_3\text{-}NH\text{-}(CH_2)_2\text{-}NH\text{-}CH_2\text{-}CH_2\text{-}CH_2\text{-}S\text{-}PO_3H$

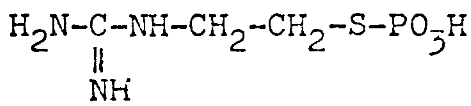,

und Zinkionen getestet. Dabei zeigte es sich, dass die zweite der oben genannten zum Stande der Technik gehörende Schwefelverbindung in Kombination mit Zinkionen und Zinkkomplexen die besten Ergebnisse liefert. Auch bei den anderen genannten Schwefelverbindungen konnte die Wirkung durch die synergistische Verabreichung der Zinkionen drastisch gesteigert werden.

## Patentansprüche

**1.** Verwendung von wasserlöslichen Zinksalzen oder wasserlöslichen Zinkkomplexen mit einer anorganischen oder organischen Säure als Wirkstoff zur Herstellung eines pharmazeutischen Präparates zur Verhütung der Schädigung lebender Zellen von Säugern durch freie Radikale, wobei vorzugsweise ein Zinksalz oder ein Zinkkomplex einer Aminocarbonsäure oder einer Hydroxycarbonsäure als Wirkstoff dieses pharmazeutischen Präparates verwendet wird.

**2.** Verwendung nach Anspruch 1, dadurch gekennzeichnet, dass die Zinksalze oder Zinkkomplexe als Wirkstoff zur Herstellung solcher pharmazeutischer Präparate zur Verhütung der Schädigung lebender Zellen durch freie Radikale eingesetzt werden, die an lebende Patienten zu verabreichende Präparate sind, welche zur Behandlung solcher durch freie Radikale ausgelöste Gewebsschäden dienen, die bei der Einwirkung einer ionisierenden Bestrahlung, bei der neuerlichen Durchblutung von Organen nach einer vorherigen Blutleere, bei der Behandlung von Patienten mit Sauerstoff, bei Gewebsalterungsvorgängen, bei der Einwirkung von freie Radikale bildenden Toxinen oder pharmazeutischen Wirkstoffen mit derartigen toxischen Nebenwirkungen zu erwarten sind oder

dass die Zinksalze oder Zinkkomplexe zur Herstellung solcher pharmazeutischer Präparate zur Verhütung der Schädigung lebender Zellen durch freie Radikale eingesetzt werden, die pharmazeutische Präparate sind, welche als Zusatz zum Perfusionsmedium bei der Durchspülung isolierter Organe verwendet werden.

**3.** Verwendung nach Patentanspruch 1 oder 2, dadurch gekennzeichnet, dass die Zinksalze oder Zinkkomplexe zur Herstellung solcher pharmazeutischer Präparate verwendet werden, die zur prophylaktischen Behandlung bei einer zu erwartenden Einwirkung von radioaktiven Stoffen oder zur Verabreichung vor einer radiodiagnostischen Untersuchung oder strahlentherapeutischen Behandlung von Krebserkrankungen eingesetzt werden, wobei die zuletzt genannten pharmazeutischen Präparate dazu dienen, das gesunde Gewebe, nicht jedoch das durch Krebs entartete Gewebe, vor der Einwirkung der ionisierenden Bestrahlung zu schützen, oder dass die Zinksalze oder Zinkkomplexe zur Herstellung solcher pharmazeutischer Präparate eingesetzt werden, die zur Verabreichung bei einer Durchblutungsstörung des Gehirns, der inneren Organe oder der peripheren Organe vor der erneuten Durchblutung vorgesehen sind, insbesondere im Zusammenhang mit einer therapeutischen Behandlung von Herzinfarkt, Gehirnarterienverschluss oder peripheren Durchblutungsstörungen, beispielsweise bei Erfrierungen,

und wobei zur Herstellung dieser pharmazeutischen Präparate vorzugsweise wasserlösliche Zinksalze oder wasserlösliche Zinkkomplexe mit einer Carbonsäure, die als weitere Substituenten Hydroxygruppen oder Aminogruppen aufweist, beispielsweise Zinkaspartat, Zinkhistidinat, Zink-orotat, oder einer schwefelhaltigen Aminosäure verwendet werden.

**4.** Verwendung gemäss einem der Patentansprüche 1 bis 4, dadurch gekennzeichnet, dass die Zinksalze oder Zinkkomplexe zur Herstellung solcher pharmazeutischer Präparate zur Verhütung der Schädigung lebender Zellen durch freie Radikale eingesetzt werden, die als weitere Komponente noch mindestens eine organische Schwefelverbindung enthalten oder dass zur Herstellung der genannten pharmazeutischen Präparate ein Zinkkomplex einer organischen Schwefelverbindung verwendet wird.

**5.** Verwendung gemäss Patentanspruch 4, dadurch gekennzeichnet, dass die genannte organische Schwefelverbindung mindestens eine freie SH-Gruppe oder eine in Form eines Thioäthers, eines Thioesters oder eines Thioharnstoffes geschützte Thiolgruppe enthält, wobei bevorzugte, Schwefel enthaltende Verbindungen Merkaptoalkylamine, Aminosäuren, die mindestens eine Thiolgruppe oder mindestens eine -S-S-Brücke aufweisen, Aminoalkylisothioharnstoffe, Thiophosphorsäureester, Thioschwefelsäureester oder Verbindungen sind, die in ihrem Molekül eine Kette aus zwei oder mehr aufeinander folgenden Schwefelatomen aufweisen.

**6.** Verwendung gemäss einem der Patentansprüche 1 bis 5, dadurch gekennzeichnet, dass die Zinksalze zur Herstellung eines durch Injektion verabreichbaren oder oral verabreichbaren oder für die topische Anwendung geeigneten pharmazeutischen Präparates verwendet werden, beispielsweise zur Herstellung einer auf die Haut aufzutragenden Creme, oder dass die Zinksalze zur Herstellung eines Präparates eingesetzt werden, welches als Zusatzstoff zu einem Perfusionsmedium zur Durchspülung isolierter Organe eingesetzt wird.

**7.** Verwendung von wasserlöslichen Zinksalzen oder wasserlöslichen Zinkkomplexen mit einer anorganischen oder organischen Säure als Wirkstoff zur Herstellung von pharmazeutischen Präparaten, welche die Wirksamkeit von pharmazeutischen Präparaten erhöhen, die organische Schwefelverbindungen enthalten, wobei eine synergistische Wirkung der genannten Zinksalze oder Zinkkomplexe mit den organischen Schwefelverbindungen auftritt.

**8.** Verwendung nach patentanspruch 7, dadurch gekennzeichnet, dass man die wasserlöslichen Zinksalze oder wasserlöslichen Zinkkomplexe zur Herstellung eines pharmazeutischen Präparates mit synergistischer Wirkung verwendet, welches als weitere Komponente noch mindestens eine organische Schwefelverbindung enthält oder dass man einen Komplex aus Zink und einer organischen Schwefelverbindung zur Herstellung des pharmazeutischen Präparates mit synergistischer Wirkung verwendet.

**9.** Verwendung gemäss Patentanspruch 7 oder 8, dadurch gekennzeichnet, dass man das Zinksalz oder den Zinkkomplex als Wirkstoff zur Erhöhung solcher organische Schwefelverbindungen enthaltender pharmazeutischer Präparate einsetzt, die zur Bekämpfung von Gewebsschäden dienen, welche durch freie Radikale ausgelöst werden, insbesondere Gewebsschäden, die bei der Einwirkung einer ionisierenden Bestrahlung oder Ultraviolettstrahlung, der neuerlichen Durchblutung von Organen nach einer vorherigen Blutleere oder der Behandlung von Patienten mit Sauerstoff auftreten können oder dass die Zinksalze oder Zinkkomplexe zur Erhöhung von solchen organische Schwefelverbindungen enthaltenden Präparaten verwendet werden, die Präparate zur Behandlung von Erkrankungen der Leber, von Haut oder Gelenksentzündungen oder alters-bedingten Schäden sind oder die prophylaktisch zu verabreichende organische Schwefelverbindungen enthaltende Präparate zur Verhinderung dieser Schäden sind.

**10.** Verwendung nach Patentanspruch 8 oder 9, dadurch gekennzeichnet, dass man das Zinksalz oder den Zinkkomplex zur Herstellung von organische Schwefelverbindungen enthaltenden pharmazeutischen Präparaten verwendet, in welchen die Schwefelverbindung mindestens eine SH-Gruppe oder eine in Form eines Thioäthers, eines Thioesters oder eines Thioharnstoffes geschützte Thiolgruppe enthält, wobei bevorzugte Schwefelverbindungen Merkaptoalkylamine, Aminosäuren, die mindestens eine Thiolgruppe oder mindestens eine -S-S-Brücke aufweisen, Aminoalkylisothioharnstoffe, Thiophosphorsäureester, Thioschwefelsäureester oder Verbindungen sind, die in ihrem Molekül eine Kette aus zwei oder mehr aufeinander folgenden Schwefelatomen aufweisen.

**11.** Verwendung nach Patentanspruch 10, dadurch gekennzeichnet, dass die in dem Präparat enthaltene Schwefelverbindung eine solche mit freier SH-Gruppe ist, die Cysteamin, Penicillamin, Aminoalkylisothioharnstoff der Formel

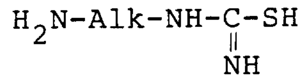

oder eine mindestens eine freie SH-Gruppe aufweisende Aminosäure, beispielsweise Cystein oder L-Glutathion ist oder dass die in dem Präparat enthaltene Schwefelverbindung eine solche mit einer geschützten SH-Gruppe ist, die eine Aminosäure mit geschützter SH-Gruppe, beispielsweise Cystin oder Methionin ist, oder dass die in dem Präparat enthaltene Schwefelverbindung eine mit einer solchen geschützten SH-Gruppe ist, die im lebenden Organismus in eine freie SH-Gruppe verwandelt wird, insbesondere S-(2-Aminoäthyl)-isothiouroniumbromid-hydrobromid der Formel

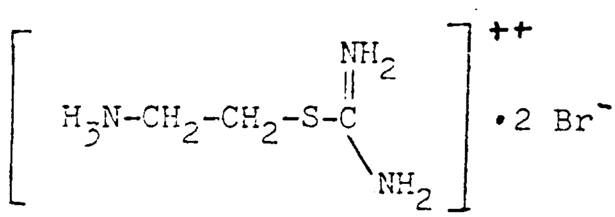

ist, oder dass die in dem Präparat enthaltene Schwefelverbindung eine solche ist, in der die SH-Gruppe durch Veresterung mit Phosphorsäure oder Schwefelsäure oder in Form einer mindestens zwei Schwefelatome umfassenden Schwefel-Schwefelbrücke geschützt ist, insbesondere die Verbindungen der Formel

$HO_2S-(CH_2)_4-S-S-S-(CH_2)_4-SO_2H$

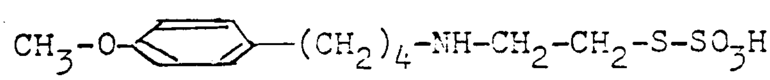

$CH_3-NH-(CH_2)_2-NH-CH_2-CH_2-CH_2-S-PO_3H$

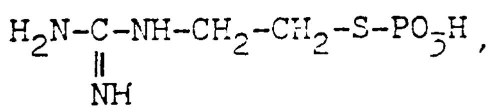

**12.** Verwendung gemäss einem der Patentansprüche 7 bis 11, dadurch gekennzeichnet, dass man als Zinksalz ein wasserlösliches Zinksalz mit einer organischen oder anorganischen Säure oder als Zinkkomplex eine Chelatverbindung aus Zink mit einer Schwefel enthaltenden organischen Verbindung verwendet, insbesondere Zinkchlorid, Zinksulfat, Zinknitrat, Zinkcarbonat oder Zinksalze mit einer Aminocarbonsäure oder Hydroxycarbonsäure, vorzugsweise Zinkaspartat, oder ein Chelat aus Zink und einer Thiolverbindung.

**13.** Verfahren zur Erhöhung der Lebensdauer isolierter, von einem Perfusionsmedium durchspülter Organe, dadurch gekennzeichnet, dass man eine Schädigung der lebenden Zellen durch freie Radikale vermindert, indem man dem Perfusionsmedium ein wasserlösliches Zinksalz oder einen Zinkkomplex zusetzt und allenfalls ausserdem eine Schwefel enthaltende organische Verbindung.

**Claims**

1. Use of water-soluble zinc salts or water-soluble zinc complexes with an inorganic or organic acid as an active agent for the manufacture of a pharmaceutical preparation for preventing damage to the living cells of mammals by free radicals, use preferably being made of a zinc salt or a zinc complex of an amino carboxylic acid or a hydroxy carboxylic acid as the active agent of said pharmaceutical preparation.

2. Use according to claim 1, **characterised in that** the zinc salts or zinc complexes are used as an active agent for the manufacture of those pharmaceutical preparations for preventing damage to living cells by free radicals which are preparations to be administered to living patients and are used to treat that tissue damage caused by free radicals which can be anticipated during the action of an ionizing radiation, during the renewed circulation of blood through organs after previous bloodlessness, during the treatment of patients with oxygen, during tissue ageing processes, during the action of toxins forming free radicals or pharmaceutical agents with toxic side effects of this kind or
in that the zinc salts or zinc complexes are used for the manufacture of those pharmaceutical preparations for preventing damage to living cells by free radicals which are pharmaceutical preparations that are used as an addition to the perfusion medium during the irrigation of isolated organs.

3. Use according to claim 1 or 2, **characterised in that** the zinc salts or zinc complexes are used for the manufacture of those pharmaceutical preparations which are used for prophylactic treatment during an anticipated action of radioactive substances or for administration prior to a radiodiagnostic examination or radiotherapeutic treatment of cancer cases, the last-named pharmaceutical preparations being used to protect the healthy tissue, but not the tissue degenerate as a result of cancer, from the action of the ionizing radiation, or that the zinc salts or zinc complexes are used for the manufacture of those pharmaceutical preparations which are intended for administration during a circulatory disturbance of the brain, of the internal organs or of the peripheral organs prior to renewed blood circulation, in particular in conjunction with a therapeutical treatment of cardiac infarction, blockage of arteries of the brain or peripheral circulatory disturbances, for example in the case of frost injuries,
and where use is made for the manufacture of said pharmaceutical preparations preferably of water-soluble zinc salts or water-soluble zinc complexes with a carboxylic acid which comprises as further substituents hydroxy groups or amino groups, for example zinc aspartate, zinc histidinate, zinc orotate, or an amino acid containing sulphur.

4. Use according to any one of claims 1 to 4, **characterised in that** the zinc salts or zinc complexes are used for the manufacture of those pharmaceutical preparations for preventing damage to living cells by free radicals which also contain as a further component at least one organic sulphur compound or that a zinc complex of an organic sulphur compound is used for the manufacture of the above-mentioned pharmaceutical preparations.

5. Use according to claim 4, **characterised in that** the above-mentioned organic sulphur compound contains at least one free SH group or one protected thiol group in the form of a thioether, a thioester or a thiourea, preferred sulphur-containing compounds being mercaptoalkyl amines, amino acids that comprise at least one thiol group or at least one -S-S- bridge, aminoalkyl isothioureas, thiophosphoric acid esters, thiosulphuric acid esters or compounds that comprise in their molecule a chain consisting of two or more successive sulphur atoms.

6. Use according to any one of claims 1 to 5, **characterised in that** the zinc salts are used for the manufacture of a pharmaceutical preparation administrable by injection or administrable orally or suitable for topical application, for example for the manufacture of a cream to be applied to the skin, or that the zinc salts are used for the manufacture of a preparation which is used as an additive to a perfusion medium for the irrigation of isolated organs.

7. Use of water-soluble zinc salts or water-soluble zinc complexes with an inorganic or organic acid as an active agent for the manufacture of pharmaceutical preparations, which enhance the effectiveness of pharmaceutical preparations that contain organic sulphur compounds, there occurring a synergic effect of the above-mentioned zinc salts or zinc complexes with the organic sulphur compounds.

**8.** Use according to claim 7, **characterised in that** the water-soluble zinc salts or water-soluble zinc complexes are used for the manufacture of a pharmaceutical preparation with synergic effect which also contains as a further component at least one organic sulphur component or that a complex of zinc and an organic sulphur compound is used for the manufacture of the pharmaceutical preparation with synergic effect.

**9.** Use according to claim 7 or 8, **characterised in that** the zinc salt or the zinc complex is used as an active agent for enhancing those pharmaceutical preparations containing organic sulphur compounds which are used for combating tissue damage that is caused by free radicals, in particular tissue damage that can occur during the action of an ionizing radiation or ultraviolet radiation, the renewed circulation of blood through organs after previous bloodlessness or the treatment of patients with oxygen, or that the zinc salts or zinc complexes are used for the enhancing of those preparations containing organic sulphur compounds which are preparations for the treatment of diseases of the liver, of the skin or arthritis or injuries caused by old age or which are preparations for preventing said injuries that contain organic sulphur compounds and are to be administered prophylactically.

**10.** Use according to claim 8 or 9, **characterised in that** the zinc salt or the zinc complex is used for the manufacture of pharmaceutical preparations containing organic sulphur compounds in which the sulphur compound contains at least one SH group or one protected thiol group in the form of a thioether, a thioester or a thiourea, preferred sulphur-containing compounds being mercaptoalkyl amines, amino acids that comprise at least one thiol group or at least one -S-S- bridge, aminoalkyl isothioureas, thiophosphoric acid esters, thiosulphuric acid esters or compounds that comprise in their molecule a chain consisting of two or more successive sulphur atoms.

**11.** Use according to claim 10, **characterised in that** the sulphur compound contained in the preparation is one containing a free SH group which is cysteamine, penicillin amine, aminoalkyl isothio urea of the formula

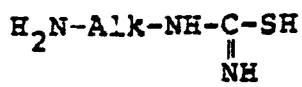

or an amino acid comprising at least one free SH group, for example cystein or L-glutathione, or that the sulphur compound contained in the preparation is one containing a protected SH group which is an amino acid with a protected SH group, for example cystein or methionine, or that the sulphur compound contained in the preparation is one with such a protected SH group which is converted in the living organism into a free SH group, in particular S-(2-aminoethyl)-isothiouroniumbromide-hydrobromide of the formula

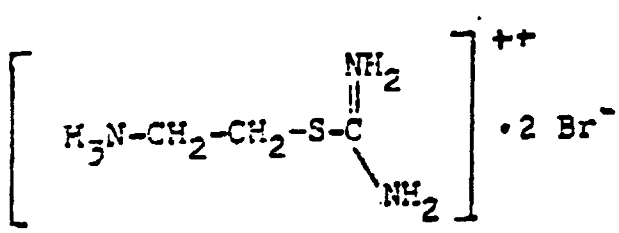

or that the sulphur compound contained in the preparation is one in which the SH group is protected by esterification with phosphoric acid or sulphuric acid or in the form of a sulphur-sulphur bridge incorporating at least two sulphur atoms, in particular the compounds with the formula

$HO_2 S-(CH_2)_4-S-S-S-(CH_2)_4-SO_2H$

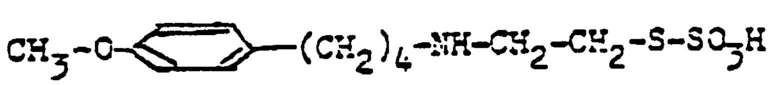

$CH_3-NH-(CH_2)_2-NH-CH_2-CH_2-CH_2-S-PO_3H$

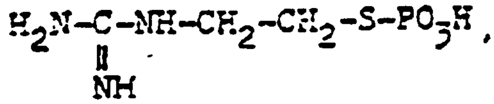

12. Use according to any one of claims 7 to 11, **characterised in that** use is made as the zinc salt of a water-soluble zinc salt with an organic or inorganic acid or as the zinc complex of a chelate compound of zinc with an organic compound containing sulphur, in particular zinc chloride, zinc sulphate, zinc nitrate, zinc carbonate or zinc salts with an amino carboxylic acid or hydroxy carboxylic acid, preferably zinc aspartate, or a chelate of zinc and a thiol compound.

13. Method for enhancing the life-span of isolated organs irrigated by a perfusion medium, **characterised in that** damage to the living cells by free radicals is reduced by adding to the perfusion medium a water-soluble zinc salt or a zinc complex and if necessary also an organic compound containing sulphur.

**Revendications**

1. Utilisation de sels de zinc solubles dans l'eau, ou de complexes de zinc solubles dans l'eau avec un acide inorganique ou organique, à titre de principe actif pour la réalisation d'une préparation pharmaceutique destinée à prévenir l'altération de cellules vivantes de mammifères par des radicaux libres, caractérisée en ce que l'on utilise, de préférence, un sel de zinc ou un complexe de zinc d'un acide aminocarboxylique, ou d'un acide hydroxycarboxylique, à titre de principe actif de cette préparation pharmaceutique.

2. Utilisation suivant la revendication 1, caractérisée en ce que les sels de zinc ou les complexes de zinc s'utilisent, à titre de principes actifs, pour la réalisation de préparations pharmaceutiques pour prévenir l' altération de cellules vivantes par des radicaux libres, qui sont des préparations à administrer à des patients vivants, qui servent au traitement d'altérations de tissus du genre de celles provoquées par des radicaux libres, altérations auxquelles il faut s'attendre lors de l'action d'un rayonnement ionisant, lors de la reprise de la circulation sanguine d'organes après une exsanguination antérieure, lors du traitement de patients par de l'oxygène, lors de processus de viellissement des tissus, lors de l'action de toxines formatrices de radicaux libres, ou lors de l'administration de principes actifs pharmaceutiques comportant de tels effets secondaires toxiques, ou en ce que

on met en oeuvre les sels de zinc ou les complexes de zinc pour la réalisation de préparations pharmaceutiques destinées à la prévention de l'altération de cellules vivantes par des radicaux libres, qui sont des préparations pharmaceutiques que l'on utilise à titre d'additif d'un milieu de perfusion au cours du rinçage, du lavage ou de l'irrigation d'organes isolés.

3. Utilisation suivant la revendication 1 ou 2, caractérisée en ce que l'on utilise les sels de zinc ou les complexes de zinc pour la réalisation de préparations pharmaceutiques du genre de celles que l'on utilise pour le traitement prophylactique lors de l'action attendue de substances radioactives, ou pour l'administration avant un examen radiodiagnostique, ou avant un traitement thérapeutique par radiation de maladies cancéreuses, où les préparations pharmaceutiques citées en dernier lieu servent à protéger le tissu sain, mais non le tissu dégénéré par le cancer, avant l'action du rayonnement ionisant, ou en ce que l'on met en oeuvre les sels de zinc ou les complexes de zinc pour la réalisation de préparations pharmaceutiques qui sont prévues pour l'administration au cours d'une perturbation de la circulation sanguine du cerveau, des organes internes, ou des organes périphériques, avant la reprise de la circulation sanguine, plus particulièrement en combinaison avec un traitement thérapeutique de l'infarctus du myocarde, de l'obstruction des artères cérébrales, ou de perturbations de la circulation sanguine périphérique, par exemple en cas de gelures,

et où pour la réalisation de ces préparations pharmaceutiques, on utilise, de préférence, des sels de zinc solubles dans l'eau, ou des complexes de zinc solubles dans l'eau avec un acide carboxylique qui présente des radicaux hydroxyle ou amino, à titre de substituants supplémentaires, par exemple

l'aspartate de zinc, l'histidinate de zinc, l'orotate de zinc, ou un aminoacide contenant du soufre.

4. Utilisation suivant l'une quelconque des revendications 1 à 4, caractérisée en ce que l'on utilise les sels de zinc, ou les complexes de zinc pour la réalisation de préparations pharmaceutiques destinées à la prévention de l'altération de cellules vivantes par des radicaux libres, préparations qui contiennent, à titre de composant supplémentaire, encore au moins un composé organique du soufre, ou en ce que, en vue de la réalisation des préparations pharmaceutiques susmentionnées, on utilise un complexe du zinc ou un composé organique du soufre.

5. Utilisation suivant la revendication 4, caractérisée en ce que le composé organique du soufre susmentionné comporte au moins un radical SH libre, ou un radical thiol protégé sous la forme d'un thioéther, d'un thioester, ou d'une thiourée, où les composés contenant du soufre, préférés, sont des mercaptoalkylamines, des aminoacides qui présentent au moins un radical thiol, ou au moins un pont -S-S-, des aminoalkylisothiourées, des esters de l'acide thiophosphorique, des esters de l'acide thiosulfurique, ou des composés qui présentent, dans leur molécule, une chaîne constituée de deux ou de plus de deux atomes de soufre qui se suivent mutuellement.

6. Utilisation suivant l'une quelconque des revendications 1 à 5, caractérisée en ce que l'on utilise les sels de zinc pour la réalisation d'une préparation pharmaceutique administrable par injection, ou administrable par la voie orale, ou convenant à l'application topique, par exemple, en vue de la préparation d'une crème à appliquer sur la peau, ou en ce que l'on met en oeuvre les sels de zinc pour la réalisation d'une préparation que l'on utilise à titre d'additif d'un milieu de perfusion pour le rinçage, le lavage ou l'irrigation d'organes isolés.

7. Utilisation de sels de zinc solubles dans l'eau, ou de complexes de zinc solubles dans l'eau avec un acide inorganique ou organique, a titre de principe actif, en vue de la réalisation de préparations pharmaceutiques qui augmentent l'activité de préparations pharmaceutiques qui contiennent des composés organiques du soufre, où se manifeste une activité synergique des sels de zinc ou des complexes de zinc susmentionnés en en combinaison avec les composés organiques du soufre.

8. Utilisation suivant la revendication 7, caractérisée en ce que l'on utilise les sels de zinc solubles dans l'eau, ou les complexes de zinc solubles dans l'eau, en vue de la réalisation d'une préparation pharmaceutique à activité synergique, qui, à titre de composant supplémentaire, contient encore au moins un composé organique du soufre, ou en ce que l'on utilise un complexe du zinc et d'un composé organique du soufre en vue de la réalisation de la préparation pharmaceutique à activité synergique.

9. Utilisation suivant la revendication 7 ou 8, caractérisée en ce que l'on met en oeuvre le sel de zinc ou le complexe de zinc, à titre de principe actif, pour l'augmentation de l'efficacité de préparations pharmaceutiques contenant des composés organiques du soufre, qui servent à lutter contre les altérations de tissus, qui sont provoquées par des radicaux libres, plus particulièrement des altérations de tissus qui peuvent se manifester lors de l'action d'un rayonnement ionisant ou lors de l'action d'un rayonnement ultraviolet, lors de la reprise de la circulation sanguine d'organes après une exsanguination antérieure, ou lors du traitement de patients par de l'oxygène, ou en ce que les sels de zinc ou les complexes de zinc s'utilisent pour l'amélioration de l'action de préparations qui contiennent des composés organiques du soufre, qui sont des préparations pour le traitement de maladies du foie, de la peau ou d'inflammations des articulations, ou d'altérations conditionnées par la vieillesse, ou qui sont des préparations contenant des composés organiques du soufre à administrer à titre prophylactique pour prévenir ces altérations.

10. Utilisation suivant la revendication 8 ou 9, caractérisée en ce que l'on utilise le sel de zinc ou le complexe de zinc pour la réalisation de préparations pharmaceutiques contenant des composés organiques du soufre, dans lesquelles le composé du soufre contient au moins un radical SH ou un radical thiol protégé sous forme d'un thioéther, d'un thioester ou d'une thiourée, où les composés du soufre préférés sont des mercaptoalkylamines, des aminoacides qui présentent au moins un pont -S-S-, des aminoalkylisothiourées, des esters de l'acide thiophosphorique, des esters de l'acide thiosulfurique, ou sont des composés qui, dans leur molécule, présentent une chaîne de deux ou de plus de deux atomes de soufre qui se suivent mutuellement.

**11.** Utilisation suivant la revendication 10, caractérisée en ce que le composé du soufre contenu dans la préparation en est un avec des radicaux SH libres, ou est la cystéamine, la pénicillamine, une aminoalkylisothiourée de la formule

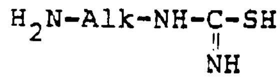

ou un aminoacide présentant au moins un radical SH libre, par exemple la cystéine ou le L-glutathion, ou en ce que le composé du soufre contenu dans la préparation en est un avec un radical SH protégé, ou est un aminoacide avec radical SH protégé, par exemple la cystéine ou la méthionine, ou en ce que le composé de soufre contenu dans la préparation en est un avec un groupe SH protégé, qui est converti dans l'organisme vivant en un radical SH libre, plus particulièrement le bromhydrate de bromure de S-(2-aminoéthyl)isothiouronium de la formule

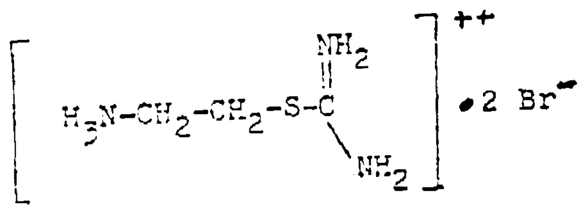

ou en ce que le composé de soufre contenu dans la préparation en est un dans lequel le radical SH est protégé par estérification avec l'acide phosphorique ou l'acide sulfurique, ou sous la forme d'au moins un pont soufre-soufre comprenant au moins deux atomes de soufre, plus particulièrement les composés des formules suivantes :

$HO_2\ S\text{-}(CH_2)_4\text{-}S\text{-}S\text{-}S\text{-}(CH_2)_4\text{-}SO_2H$

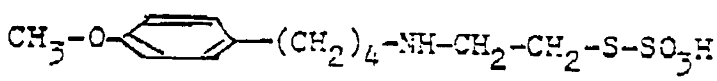

$CH_3\text{-}NH\text{-}(CH_2)_2\text{-}NH\text{-}CH_2\text{-}CH_2\text{-}CH_2\text{-}S\text{-}PO_3H$

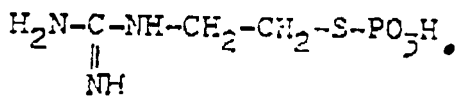

**12.** Utilisation suivant l'une quelconque des revendications 7 à 11, caractérisée en ce que l'on utilise, a titre de sel de zinc, un sel de zinc soluble dans l'eau d'un acide organique ou inorganique, ou, à titre de complexe de zinc, un composé du type chélate constitué de zinc et d'un composé organique contenant du soufre, plus particulièrement le chlorure de zinc, le sulfate de zinc, le nitrate de zinc, le carbonate de zinc, ou des sels de zinc d'un acide aminocarboxylique ou d'un acide hydroxycarboxylique, de préférence l'aspartate de zinc, ou un chélate constitué de zinc et d'un composé du type thiol.

**13.** Procédé pour augmenter la durée de vie d'organes isolés, rincés, lavés ou irrigués par un milieu de perfusion caractérisé en ce que l'on réduit l'altération des cellules vivantes par des radicaux libres pour autant que l'on ajoute un sel de zinc soluble dans l'eau ou un complexe de zinc et, en outre, au besoin, un composé organique contenant du soufre.

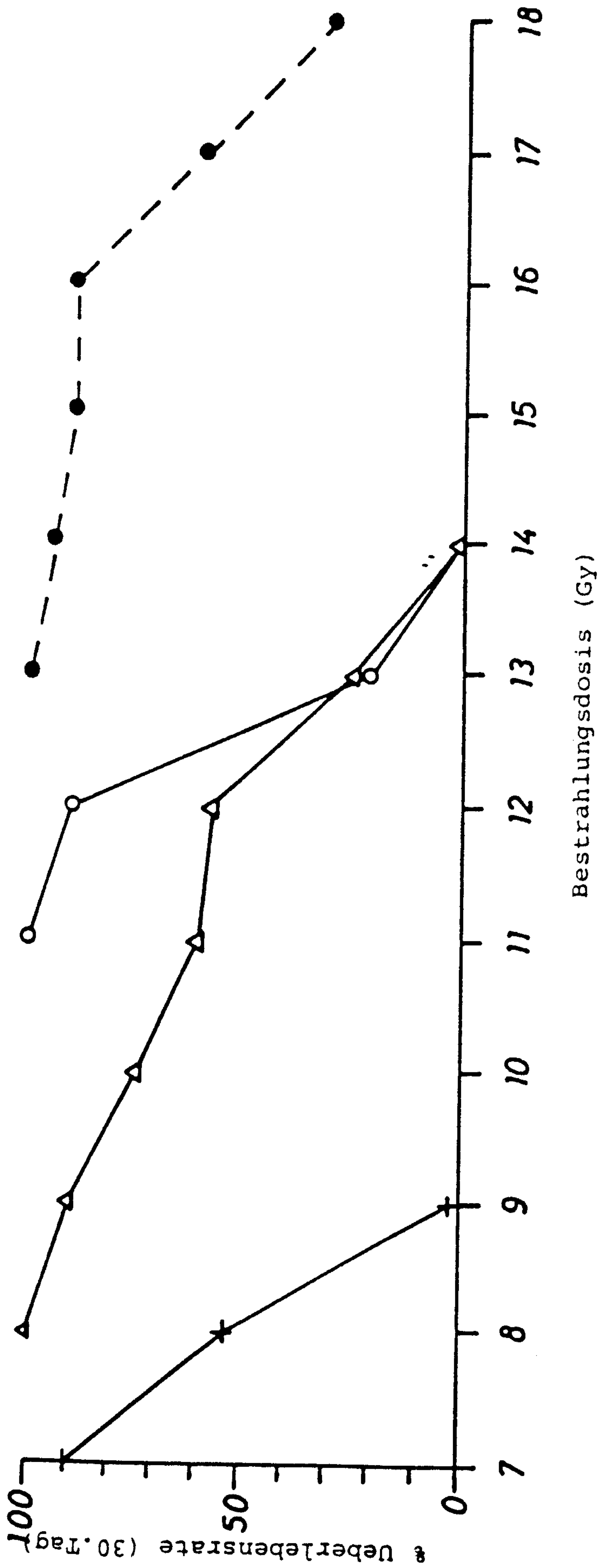
% Ueberlebensrate (30.Tag)
100
50
0
7
8
9
10
11
12
13
14
15
16
17
18
Bestrahlungsdosis (Gy)